(19) Europäisches Patentamt
European Patent Office
Office européen des brevets

(11) Publication number: **0 479 161 A1**

# (12) EUROPEAN PATENT APPLICATION

(21) Application number: **91116559.5**

(22) Date of filing: **27.09.91**

(51) Int. Cl.$^{5}$: **C07D 263/56**, C07D 277/66, C07D 235/18, C07D 263/60, C07D 413/04

(30) Priority: **01.10.90 US 591308**

(43) Date of publication of application: **08.04.92 Bulletin 92/15**

(84) Designated Contracting States: **DE FR GB**

(71) Applicant: **EASTMAN KODAK COMPANY**
**343 State Street**
**Rochester, New York 14650-2201(US)**

(72) Inventor: **Perry, Robert James**
**c/o EASTMAN KODAK COMPANY, 343 State Street**
**Rochester, New York 14650-2201(US)**

(74) Representative: **Brandes, Jürgen, Dr.Rer.Nat. et al**
**Wuesthoff & Wuesthoff, Patent- und Rechtsanwälte, Schweigerstrasse 2**
**W-8000 München 90(DE)**

## (54) Synthesis of heterocyclic compounds.

(57) A new synthetic method is reported in which 2-arylbenz(ox,imid,thi)azoles can be prepared by the palladium catalyzed condensation of aromatic halides with o-amino(phenol, aniline, thiophenol)s followed by dehydrative cyclization. This method is tolerant of a wide variety of functional groups on either aromatic ring and gives good to excellent yields of products.

EP 0 479 161 A1

The present invention is directed to a synthetic method for the formation of heterocyclic compounds. The compounds that are formed are useful as anesthetics, analgesics, virus inhibitors, metal coordinators and fungicides.

Arylbenz(ox, imid, thi)azoles are commonly made from the condensation of an aromatic carboxylic acid (or derivative) and an o-amino(phenol, aniline, thiophenol). Initial reaction between these two compounds results in the formation of an amide intermediate. This intermediate is then subjected to dehydrative cyclization.

One limitation to this method is the unavailability of certain aryl carboxylic acid reagents. Use of the corresponding nitrile, selenoamide or N-ethoxycarbonylthioamide provides alternate routes to the desired product but suffers from long syntheses and/or toxic by-products. In addition, the selection of carboxylic acid derivatives is limited and the reaction conditions are such that some functional substituents are difficult to make. Thus, the problem to be overcome is how to provide an alternative synthetic method for producing these useful compounds that avoids these difficulties of previous methods.

In accordance with the present invention, there is provided a method for the production of heterocyclic organic compounds comprising the steps of:

a) condensing an aromatic halide with an orthoamino(phenol, aniline, thiophenol) in the presence of carbon monoxide, a base and a palladium catalyst; and

b) causing the intermediate formed in step a) to undergo ring closure to give the desired heterocycle.

The process of the invention can be illustrated by the following reaction scheme:

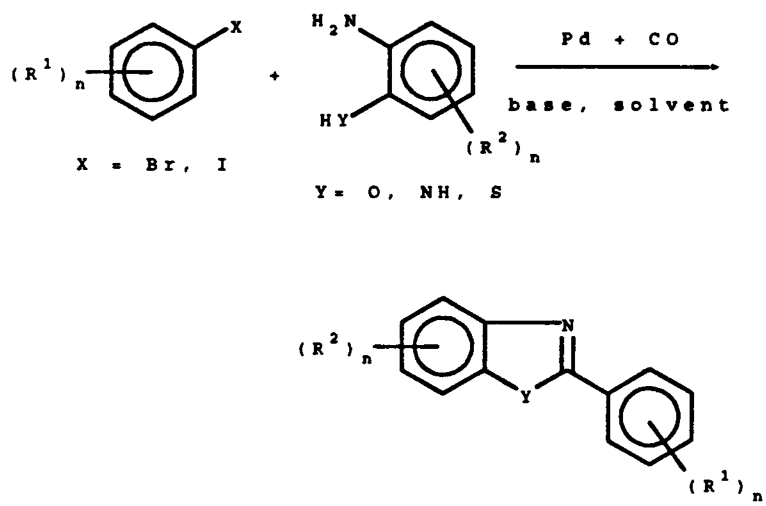


($R^1$, $R^2$ and n are described in more detail in the detailed description below.)

As noted above, the invention involves the reaction of an optionally substituted aromatic halide with an optionally substituted o-amino(phenol, aniline, thiophenol). The aromatic halide is preferably represented by the formula:

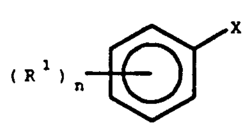

wherein X is bromide or iodide; and

$R^1$ is selected from the group consisting of alkyl, alkoxy, aryloxy (wherein the alkyl group preferably has from 1 to 22 carbon atoms and the aryl group has from 6 to 10 carbon atoms), disubstituted amino, cyano, chloro, aryl, acetyl, nitro, carboxylic acid (and derivatives such as esters), sulfone and amide. There can be up to four $R^1$ groups on the rings, thus, n can be an integer from 0 to 4.

Other useful aromatic halides include compounds having a heteroatom in the ring structure. Typical compounds of this type include:

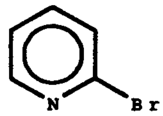 , 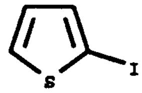 , 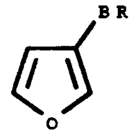 and 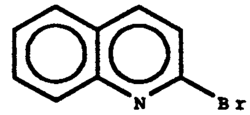

The o-amino phenol can be represented by the formula:

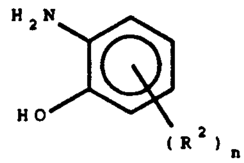

wherein $R^2$ and n are defined as for $R^1$ and n above.

A variety of benzoxazoles have been made by the process of the present invention. These compounds are listed below in Table 1. For compounds 1-10, the indicated aromatic halide was reacted with o-aminophenol. For compounds 11-15, the indicated o-aminophenol was reacted with iodobenzene.

Table 1

| Cpd. | Aromatic halide | Product |
|---|---|---|
| (1) | 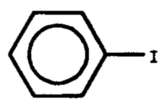 | 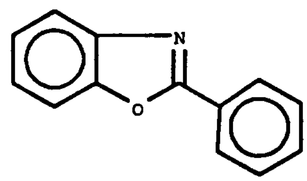 |
| (2) | 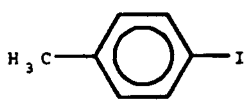 | 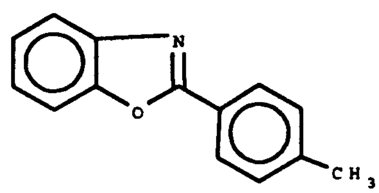 |

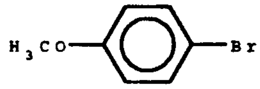
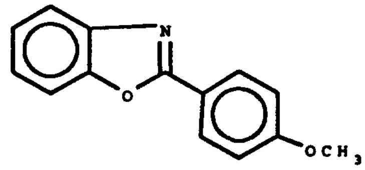

(3)

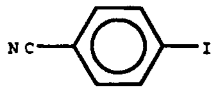
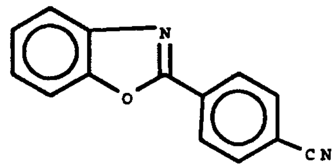

(4)

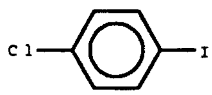
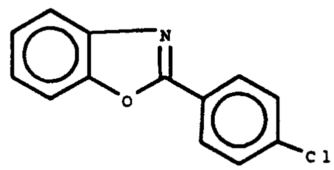

(5)

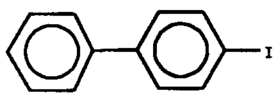
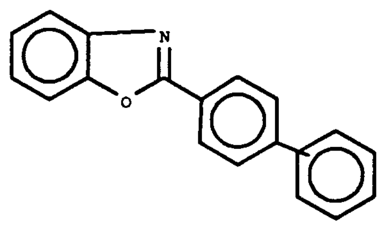

(6)

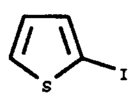
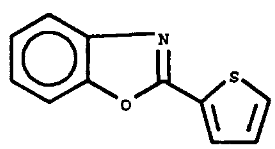

(7)

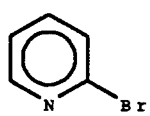
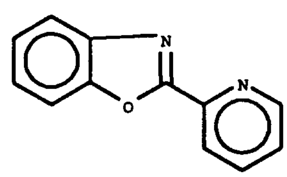

(8)

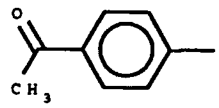

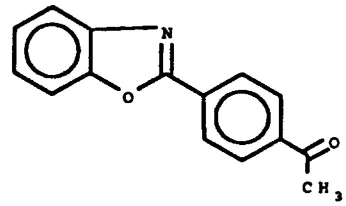

(9)

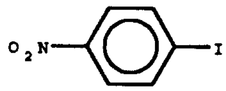

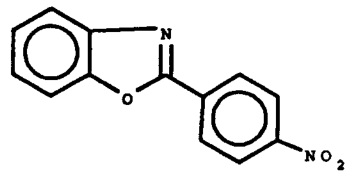

(10)

Cpd. Aminophenol Product

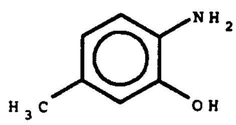

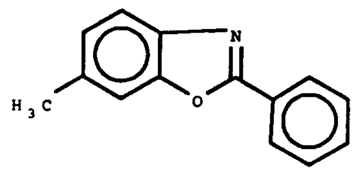

(11)

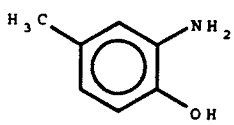

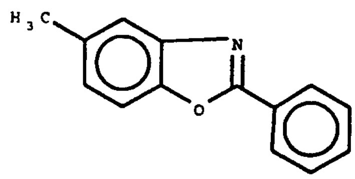

(12)

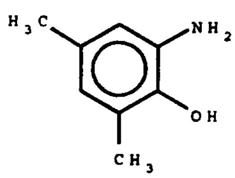

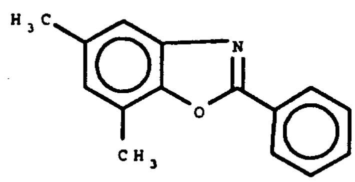

(13)

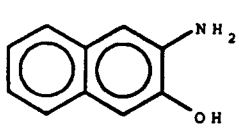

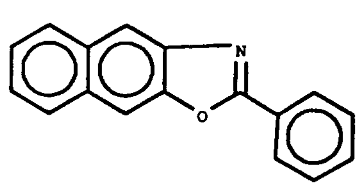

(14)

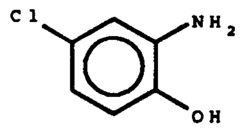 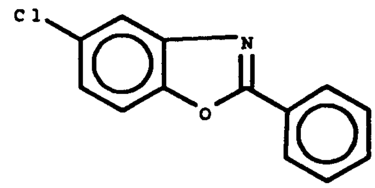

(15)

A variety of benzimidazoles and benzthiazoles have also been made by the method of the present invention. These compounds are listed in Table 2 below:

Table 2

| Cpd. | Aromatic Halide | Amino Cpd. | Product |
|---|---|---|---|
| (16) | 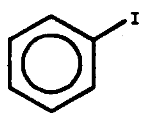 | 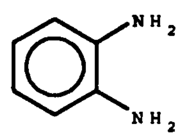 | 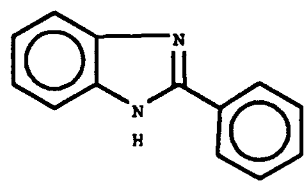 |
| (17) | 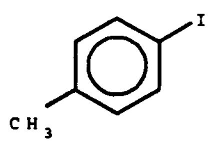 | 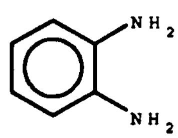 | 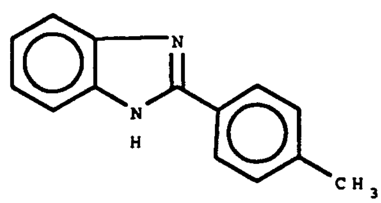 |

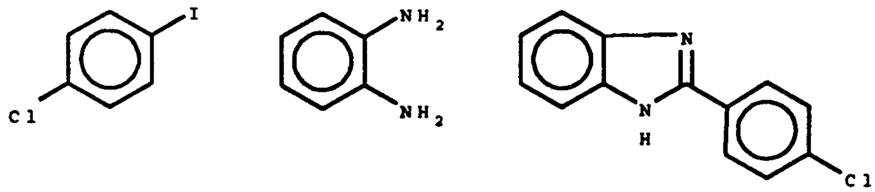

(18)

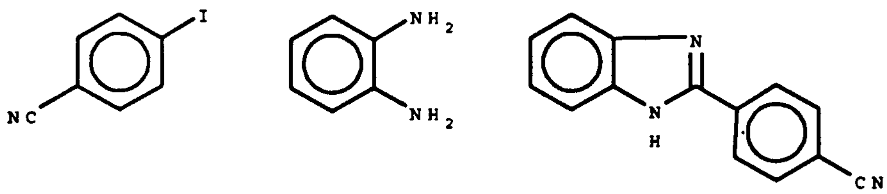

(19)

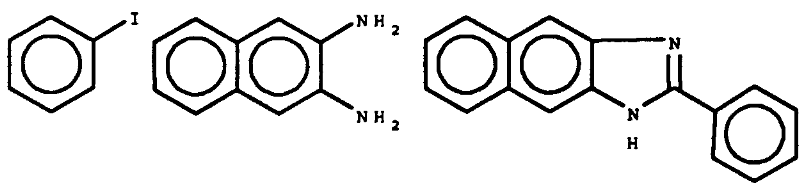

(20)

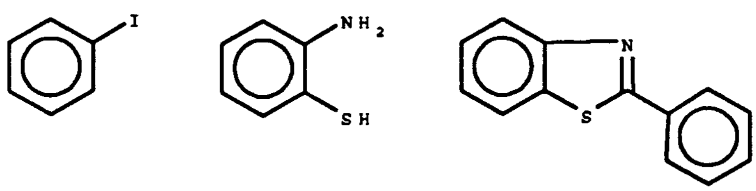

(21)

As can be seen by the above description, a wide variety of halo and amino reactants can be used in the processes of this invention. Preferably, such reactants are "stable" under the reaction conditions employed, that is, they do not decompose to an unacceptable extent during the process of this invention. The organic materials used in this invention are also "suitably reactive", that is, the process of this invention without entering into an unacceptable amount of undesirable side reaction(s). Thirdly, the organic reactants used in this invention should be "sterically suitable", that is, that they not be so bulky as to unduly retard the reaction by steric hindrance. Examples of such reactants have been given above.

The amine and halide reactants are contacted with carbon monoxide. The CO may be at atmospheric pressure or at a higher pressure. Carbon monoxide pressures in the range of from 1 to 200 atmospheres or higher can be used in the process.

Pressures lower than atmospheric can be used if desired, but generally do not confer any advantage.

It is convenient to add an excess of carbon monoxide to the reaction zone. The excess of CO need not be measured; one may merely pressurize the vessel with CO to the desired reaction pressure.

When one of the organic reactants is used in excess, it is preferably used in an amount of from 1.001 to

5 times the molar amount required by stoichiometry.

The process of this invention is conducted in the presence of a liquid reaction medium to facilitate contacting the reactants. A wide variety of organic compounds can be used for this purpose so long as the reaction medium is "inert", that is, does not enter into the reaction in an undesired way. It is preferred that the reaction medium dissolve the reactant(s) to an appreciable extent. A preferred solvent of this type is tetrahydrofuran or diglyme (2-methoxyethyl ether), or glyme (1,2-dimethoxy ethane). A dipolar aprotic solvent is preferentially employed. Such solvents are characterized by the lack of acidic, easily abstractable hydrogens and are highly polar molecules. Typical dipolar aprotic solvents are dimethyl formamide, dimethylacetamide, N-methylpyrrolodinone, dimethylsulfoxide, hexamethylphosphoramide, and the like. The amount of liquid reaction medium is not critical. Generally, one uses enough medium to facilitate the reaction. There is no real upper limit on the amount of reaction medium employed. However, practical limits are imposed by the size of the reaction vessel, the ease of separation of product(s) from the reaction medium, cost, and similar considerations. Generally, the amount of liquid reaction medium employed is within the range of from 0.1 to 800 volumes based on the volume of halo aromatic employed.

The process of this invention is conducted in the presence of a catalyst. The catalyst is preferentially a palladium compound, where palladium is present in the zero valent or divalent state. The palladium catalysts generally have one or more ligands bonded to the palladium atom(s) by ionic or covalent bonds. Simple palladium salts such as PdX'2 wherein X' is Cl, Br or I can be used. Other representative palladium catalysts are listed in Table 3 below:

**Table 3**

**Palladium Catalysts**

| | |
|---|---|
| $Pd^{+2}$ | |
| $PdX_2$ | X = Cl, Br, I |
| $PdX_2L_2$ | X = Cl, Br, I |
| | L = $R_3P$, where R = alkyl or aryl |
| $Pd(OAc)_2$ | OAc = acetate |
| $Pd(OAc)_2L_2$ | OAc = acetate |
| $PdCl_2(RCN)_2$ | R = $CH_3$, Phenyl |
| $PhPdXL_2$ | X = Br, I |
| $PdCl_2(COD)_2$ | COD = cis,cis-1,5-cyclooctadiene and; |
| $Pd(acac)_2$ | acac = 2,4-pentanedionate |
| $PdCl_2(DPPE)$ | DPPE= 1,2-bis(diphenylphosphino)ethane |

$Pd^{(0)}$

PdL L= $R_3P$ where R = alkyl or aryl and;

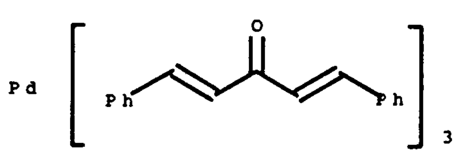

$Pd(DPPE)_2$

A catalytic amount of catalyst is employed. By "catalytic amount" is meant an amount of catalyst which catalyses the reaction to the desired extent. Generally, the amount of catalyst is at least 0.05 mole percent based on the amount of aromatic halide. There is no real upper or lower limit on the amount of catalyst, this being defined by secondary conditions such as cost and ease of separation of the catalyst from products and unreacted reactants. A preferred catalytic amount is from 0.005 to 0.20 moles per mole of aromatic halide, more preferably from .02 to .10 mole per mole of halide reactant.

The process of this invention is preferably conducted in the presence of a base to neutralize by-product hydrogen halide. The base may be a tertiary amine such as tributylamine, 2,6-lutidine, 1,8-diazobicyclo-[5,4,0]-7-undecene (DBU) or have the formula:

$NR_3$

wherein each R is independently selected from lower alkyl groups having from 2 to 6 carbon atoms. The base may be immobilized on a cross-linked polymer such as cross-linked poly(vinylpyridine) beads. Alternatively, the base may be another type of basic substance which does not react with the reactants, for example, a metal carbonate such as K2CO3 or a metal hydroxide such as $Ca(OH)_2$. Generally, one employs at least enough base to react with the by-product HX produced. An excess can be used, if desired. As with the reactants, solvents and catalysts, a skilled practitioner will recognize that the exact structure of the base is not critical, and the examples of compounds set forth above are merely illustrative and not-limiting examples of materials that can be used in this invention. A skilled practitioner will recognize that other materials can be substituted in this invention to achieve similar results. For the benzimidazole reaction, 2,6-dimethylpyridine is the preferred base because it allow for monoamidation.

The process of this invention is preferably conducted at a temperature within the range of from about ambient to 250°C. A preferred temperature range is from 60°C to 160°C. A skilled practitioner will recognize that the reaction temperature is not critical, and that temperatures outside this range can be employed, if desired. Generally, one selects a reaction temperature which affords a reasonable rate of reaction and which does not give an undue amount of decomposition of products or reactants. The reaction time is not a truly independent variable, but is dependent at least to some extent based on the other reaction parameters selected such as reactivity of the reactants, activity, and amount of catalyst, reaction temperature, pressure, and similar variables. Generally speaking, reaction times within the range of from 0.1 to 100 hours are used.

The second step in the process of the invention involves the cyclization of the intermediate that is formed by the reaction of the aromatic halide with the o-aminophenol, amine or thiophenol. Conventional methods such as thermal cyclization in the presence of an acid such a hydrochloric acid as described in Cassidy, P. "Thermally Stable Polymers", Marcel Decker, Inc. New York, 1980, pg 154 are useful. This HCl method of cyclization is referred to in the Examples as "Method A".

In a preferred method, the initial amide is prepared in toluene and after carbonylation and coupling are complete, methanesulfonic or p-toluenesulfonic acid is added and the solution refluxed to remove water. This results in much cleaner and faster reactions from which high yields of the benzoxazole product can be easily isolated. The process is completed by neutralization with $NH_4OH$, digestion with hot toluene then concentration of the toluene fractions and chromatography through a short column of silica gel to give product. In most of these reactions small amounts of the amide-esters were formed as determined by gas chromotography and mass spectrometry. These were easily removed when the crude product was "filtered" through a short column of silica gel. This procedure also removes the catalyst and any base, for example, 1,8-diazobicyclo[5,4,0]-7-undecene (DBU), remaining. Most of the salts that formed from the amidation reaction are left behind as a sludge after the hot toluene extraction. This preferred method of cyclization is referred to in the Examples as "Method B".

All reactions were monitored on an gas chromatograph using a 15 m, 0.1 $\mu$m DB-5 column (0.32 mm i.d.) and a flame ionization detector. Helium flow rate through the column was 4.0 mL/min. The G.C. parameters employed for analysis were as follows: injection port, 300°C; detector, 350°C; temperature ramp from 50°C (hold 1 min) to 300°C (hold 10 min) at 20°C/min. Proton NMR spectra and 13C NMR spectra were recorded on a spectrometer using chloroform as an internal standard. Fourier transform infrared spectra were recorded on a spectrometer as KBr pellets. Chromatography was performed on a radial layer chromatographic device using 4 mm PF-254 silica gel plates.

The following examples are presented for a further understanding of the invention:

The initial amide forming reaction was performed in a 3 oz. pressure reaction vessel (containing a stir-bar) fitted with a pressure gauge, a pressure release valve, a gas inlet and a straight ball valve for degassing and sample withdrawal. All reactions were run at 0.33 M in N,N-dimethylacetamide (DMAc) at 120°C under 7.38 kg/sq.cm CO using 3% $PdCl_2L_2$ and 6%$PPh_3$ as the catalyst system unless otherwise noted.

Dehydrative cyclizations were accomplished by transferring the contents of the pressure reactor to a 3-neck, round-bottom flask equipped with a stir bar, a condenser and a gas inlet for argon and then adding the appropriate reagent as described later.

DMAc was freshly distilled from BaO or purchased (anhydrous) and toluene was distilled from

sodium/benzophenone ketyl. Triphenylphosphine was recrystallized from hexanes, 4-iodobiphenyl was recrystallized from ethanol, DBU was fractionally distilled under reduced pressure and o-aminophenol was recrystallized from ethanol. Other chemicals were used as purchased.

Example 1 Preparation of 2-phenylbenzoxazole, (Table 1 compound 1) (Method A)

A pressure reaction vessel was charged with o-aminophenol (540 mg, 4.95 mmol), iodobenzene (540 µL, 4.95 mmol), $PdCl_2L_2$ (35 mg, 0.05 mmol), $PPh_3$ (26 mg, 0.10 mmol) and DMAc (15 mL). The contents were deoxygenated with argon then placed under 2.46 kg/sq.cm CO and stirred at 120°C until all reagent had dissolved. Then the pressure was released and DBU (890 µL, 5.94 mmol) was added by syringe through the ball valve. The reactor was charged to 7.38 kg/sq.cm CO and stirred for 0.5 h after which time a small aliquot was removed. GC analysis indicated no iodobenzene or aminophenol remained but a single peak for the amidephenol was present. 1H NMR ($CDCl_3$) δ8. 63 (br s, 1), 8.15 (br s, 1), 7.92 (d, J = 7.0 Hz, 2), 7.61 (m, 1), 7.53 (m, 1), 7.20 (m, 3), 7.09 (d, J = 7.8 Hz, 1), 6.93 ( t, J = 7.0 Hz, 1). IR($CDCl_3$) 3415, 3060, 1645, 1610, 1590, 1575, 1545, 1455, 1440, 1365, 1285, 1240, 750, 700 $cm^{-1}$.

The contents of the vessel were transferred to a flask, diluted with 10 mL DMAc and 5 drops of concentrated HCl added. The solution was brought to reflux and allowed to react for 16 h. GC analysis indicated that no intermediate product remained, only ring-closed material was present. The contents of the flask were concentrated in vacuo, diluted with ether (100 mL) and washed with water (3 x 50 mL). The ether layer was dried over $MgSO_4$, concentrated in vacuo and purified by using 3:1 hexanes:ethyl acetate to give 604 mg (63%) product as white crystalline solid. mp 101-103°C, (literature mp 102-104°C). 1H NMR ($CDCl_3$) δ 8.28 (m, 2), 7.81 (m, 1), 7.61(m, 1), 7.55 (m, 3), 7.37 (m, 2). 13C{1H} NMR ($CDCl_3$) d 163.0, 150.8, 142.0, 131.4, 128.8, 127.5, 127.0, 125.0, 124.5, 119.9, 110.5. IR(KBr) 3070, 1625, 1555, 1460, 1245, 1060, 750, 705, 690 $cm^{-1}$.

Example 2 2-phenylbenzoxazole, (Table 1 compound 1) (Method B)

A pressure vessel was charged with o-aminophenol (976 mg, 8.94 mmol), iodobenzene (1.00 mL, 8.94 mmol), $PdCl_2L_2$ (94 mg, 0.13 mmol) and toluene (27 mL). The contents were deoxygenated with argon then placed under 2.46 kg/sq.cm CO and stirred at 120°C until all reagents had dissolved. Then the pressure was released and DBU (1.60 mL, 10.7 mmol) was added by syringe through the ball valve. The reactor was charged to 7.38 kg/sq.cm CO and stirred for 75 minutes. The reaction mixture was transferred to a round bottom flask (several mL of dichloromethane were used to rinse the reaction vessel), diluted with more toluene treated with methanesulfonic acid (3 mL) and refluxed. A trap collected the water of dehydration. After 2 hours, the amide had completely cyclized. The reaction mixture was neutralized with $NH_4OH$ (4 mL). The organic solution was decanted and the residue extracted with hot toluene (2 x 30 mL). The toluene extracts were combined, concentrated in vacuo and filtered through a plug of silica gel (eluting with toluene). A pale yellow solid was isolated which was further purified by sublimation at 80°C / 1.7 torr to give 1.44 g product (86%).

Example 3 2-(p-tolyl)benzoxazole, (Table 1 compound 2) (Method A)

As described above, o-aminophenol (540 mg, 4.95 mmol), 4-iodotoluene (1.074 g, 4.95 mmol), $PdCl_2L_2$ (36 mg, 0.05 mmol), $PPh_3$ (26 mg, 0.10 mmol), DMAc (15 mL) and DBU (890 µL, 5.94 mmol) were allowed to react together for 1 h under 7.38 kg/sq.cm CO then transferred to a flask, diluted with DMAc (5 mL) and heated to 165°C in the presence of 5 drops conc HCl. After 16 h the reaction mixture was concentrated in vacuo then purified with ethyl acetate to give 825 mg (80%) product as a crystalline solid which was recrystallized from ethanol. mp 114-115°C. literature mp 114.5°C. 1H NMR ($CDCl_3$) d 8.14 (d, J = 8.2 Hz, 2), 7.75 (m, 1), 7.56 (m, 1), 7.33 (m, 2), 7.32 (d, J = 8.0 Hz, 2), 2.42 (s, 3). 13C{1H} NMR ($CDCl_3$) δ 163.1, 150.6, 142.1, 142.0, 129.6, 127.5, 124.8, 124.4, 124.3, 119.8, 110.4, 21.6. IR(KBr) 3060, 3035, 2920, 1625, 1555, 1500, 1455, 1240, 1175, 1060, 820, 745, 500 $cm^{-1}$.

Example 4 2-(4-methoxyphenyl)benzoxazole, (Table 1 compound 3) (Method B)

As described above, o-aminophenol (873 mg, 8.0 mmol), 4-iodoanisole (1.872 g, 8.0 mmol), $PdCl_2L_2$ - (84 mg, 0.12 mmol), toluene (25 mL) and DBU (1.44 mL, 9.6 mmol) were allowed to react together for 2 h under 7.38 kg/sq.cm CO then transferred to a flask, diluted with toluene and heated to reflux in the presence of p-toluenesulfonic acid (p-TSA, 6.0 g) for 2 h. The usual workup afforded 1.48 g (82%) product.

mp 102-104 °C. literature mp 105 °C. 1H NMR ($CDCl_3$) δ 8.23 (d, J = 8.6 Hz, 2), 7.76 (m, 1), 7.57 (m, 1), 7.35 (m,2), 7.06 (d, J = 8.6 Hz, 2). 13C{1H} NMR ($CDCl_3$) d 162.9, 162.2, 150.6, 142.2, 129.3, 124.5, 124.4, 119.6, 119.5, 114.3, 110.3, 55.4. IR(KBr) 3040, 2940, 1610, 1605, 1505, 1455, 1255, 1245, 1170, 1020, 830, 740, 725 $cm^{-1}$.

Example 5 2-(4-cyanophenyl)benzoxazole, (Table 1 compound 4) (Method A)

As described above, o-aminophenol (540 mg, 4.95 mmol), 4-iodobenzonitrile (1.130 g, 4.95 mmol), $PdCl_2L_2$ (36 mg, 0.05 mmol), $PPh_3$ (26 mg, 0.10 mmol), DMAc (15 mL) and DBU (890 µL, 5.94 mmol) were allowed to react together for 1 h under 7.38 kg/sq.cm CO then transferred to a flask, diluted with DMAc (5 mL) and heated to 165 °C in the presence of 10 drops conc HCl. After 36 h the reaction mixture was concentrated in vacuo then purified with 3:1 hexanes:ethyl acetate to give 610 mg (56%) product as a slightly yellow solid mp 201-205 °C. literature mp 204-207 °C. 1H NMR ($CDCl_3$) δ 8.36 (d, J = 8.5 Hz, 2), 7.82 (d, J = 8.4 Hz, 2), 7.82 (m, 1), 7.61 (m, 1), 7.42 (m, 2). 13C{1H} NMR ($CDCl_3$) d 150.9, 141.9, 138.7, 132.7, 131.1, 128.0, 126.1, 125.1, 120.6, 118.2, 114.7, 110.9. IR(KBr) 3100, 3060, 2240, 1615, 1550, 1495, 1455, 1415, 1245, 1060, 845, 750, 550 $cm^{-1}$.

Example 6 2-(4-chlorophenyl)benzoxazole, (Table 1 compound 5) (Method A)

As described above, o-aminophenol (540 mg, 4.95 mmol), 4-chloroiodobenzene(1.180g, 4.95 mmol), $PdCl_2L_2$ (36 mg, 0.05 mmol), $PPh_3$ (26 mg, 0.10 mmol), DMAc (15 mL) and DBU (890 µL, 5.94 mmol) were allowed to react together for 1 h under 7.38 kg/sq.cm CO then transferred to a flask, diluted with DMAc (5 mL) and heated to 165 °C in the presence of 10 drops conc HCl. After 36 h the reaction mixture was concentrated in vacuo then purified with 3:1 hexanes:ethyl acetate to give 739 mg (54%) product as a crystalline solid. mp 147-148 °C. literature mp 149 -150 °C. 1H NMR ($CDCl_3$) δ 8.18 9 (d, J = 8.6 Hz, 2), 7.76 (m, 1), 7.57 (m, 1), 7.48 (d, J = 8.5 Hz, 2), 7.36 (m, 2). 13C{1H} NMR ($CDCl_3$) d 161.8, 150.7, 142.0, 137.7, 129.2, 128.8, 125.6, 125.3, 124.7, 120.0, 110.6. IR(KBr) 3060, 1620, 1600, 1480, 1455, 1405, 1245, 1090, 1060, 1015, 835, 740, 500 $cm^{-1}$.

Example 7 2-(4-biphenylyl)benzoxazole, (Table 1 compound 6) (Method B)

As described above, o-aminophenol (655 mg, 6.0 mmol), 4-iodobiphenyl (1.681 g, 6.0 mmol), $PdCl_2L_2$ - (63 mg, 0.09 mmol), toluene (18 mL) and DBU (1.08 mL, 7.2 mmol) were allowed to react together for 2 h under 7.38 kg/sq.cm CO then transferred to a flask, diluted with toluene and heated to reflux in the presence of methanesulfonic acid (1 mL) for 4 h. The usual workup afforded 1.99 g crude product. The crude material was again passed through a short column of silica gel (eluting with benzene) to give 1.55 g pure product (97%). mp 136 - 138 °C. literature mp 138 °C. 1H NMR ($CDCl_3$) δ 8.31 (d, J = 8.2 Hz, 2), 7.77 (m, 3), 7.65 (m, 2), 7.61 (m, 1), 7.48 (m, 2), 7.39 (m, 3). 13C {1H} NMR ($CDCl_3$) d 162.7, 150.7, 144.1, 142.1, 139.9, 128.9, 128.0, 127.5, 127.1, 125.9, 125.0, 124.5, 119.9, 110.5. IR(KBr) 3060, 3040, 1620, 1570, 1480, 1450, 1405, 1245, 1060, 845, 740, 700 $cm^{-1}$.

Example 8 2-(2-thienyl)benoxazole, (Table 1 compound 7) (Method A)

As described in the general experimental section above o-aminophenol (390 mg, 3.58 mmol), 2-iodothiophene (395 µL, 3.58 mmol), $PdCl_2L_2$ (38 mg, 0.053 mmol), $PPh_3$ (28 mg, 0.106 mmol), DMAc (10.8 mL) and DBU (640 µL, 4.28 mmol) were allowed to react together for 0.5 h under 7.38 kg/sq.cm CO then transferred to a flask, diluted with CHP (20 mL) and heated to 240 °C in the presence of 10 drops of conc HCl. After 4.5 h only product was seen by GC. The reaction mixture was concentrated in vacuo then purified with 4:1 hexanes:ethyl acetate to give 370 mg ( 51%) product as a crystalline solid which was recrystallized from ethanol/water to give white platelets. mp 102-103 °C. literature mp 103-105 °C. 1H NMR ($CDCl_3$) δ 7.92 (d, J = 8.6 Hz, 2), 7.75 (m, 1), 7.55 (m, 1), 7.35 (m, 1), 7.20 (d, J = 4.8, 3.9 Hz, 1). 13C{1H} NMR ($CDCl_3$) d 158.0, 150.4, 141.9, 130.2, 129.9, 129.6, 128.2, 125.0, 124.7, 119.7, 110.4. IR(KBr) 3080, 3060, 1615, 1570, 1500, 1450, 1420, 1245, 1230, 1010, 850, 760, 745, 720 $cm^{-1}$.

Example 9 2-(2-pyridyl)benoxazole, (Table 1 compound 8) (Method B)

As described above, o-aminophenol (916 mg, 8.39 mmol), 2-bromopyridine (800 mL, 8.39 mmol), $PdCl_2L_2$ (88 mg, 0.12 mmol), $PPh_3$ (66 mg, 0.25 mmol), toluene (26 mL) and DBU (1.50 mL, 10.07 mmol)

were allowed to react together for 2.5 h under 7.73 kg/ sq.cm CO then transferred to a flask, diluted with toluene and heated to reflux in the presence of p-toluenesulfonic acid (p-TSA, 6.0 g) for 2 h. GC analysis of the reaction mixture indicated incomplete conversion to ring-closed product so additional p-TSA was added (2.0 g). After 17 h the usual workup afforded 1.36 g (82%) product. mp 107-108 °C. literature mp 108 °C. 1H NMR ($CDCl_3$) δ 8.75 (d, J = 4.5 Hz, 1), 8.28 (d, J = 7.8 Hz, 1), 7.79 (m, 2), 7.60 (m, 1), 7.34 (m, 3). 13C {1H} NMR ($CDCl_3$) d 161.2, 150.1, 145.8, 141.5, 136.9, 126.5, 125.9, 125.5, 124.7, 123.2, 120.4, 111.0. IR-(KBr) 3070, 1585, 1550, 1455, 1440, 1350, 1240, 1080, 740 $cm^{-1}$.

Example 10 2-(4-acetylphenyl)benzoxazole, (Table 1 compound 9) (Method A)

As described above, o-aminophenol (540 mg, 4.95 mmol), 4-bromoacetophenone (985 mg, 4.95 mmol), $PdCl_2L_2$ (36 mg, 0.05 mmol), $PPh_3$ (26 mg, 0.10 mmol), DMAc (15 mL) and DBU (890 µL, 5.94 mmol) were allowed to react together for 1 h under 7.38 kg/sq.cm CO then transferred to a flask, diluted with DMAc (5 mL) and heated to 165 °C in the presence of 10 drops conc HCl. After 72 h the reaction mixture was concentrated in vacuo, isolated with 3:1 hexanes:ethyl acetate and recrystallized from ethanol to give 632 mg (54%) product as a crystalline solid mp 168-170 °C. 1H NMR ($CDCl_3$) δ 8.42 (d, J = 8.4 Hz, 2), 8.17 (d, J = 8.4 Hz, 2), 7.87 (m, 1), 7.67 (m, 1), 7.46 (m, 1), 2.74 (s, 3). 13C(1H) NMR ($CDCl_3$) d 197.2, 161.6, 150.9, 141.9, 138.8, 131.0, 128.7, 125.7,124.8, 120.3, 110.7, 26.7. IR(KBr) 3060, 2940, 1680, 1620, 1600, 1555, 1450, 1410, 1360, 1260, 1055, 845, 750 $cm^{-1}$. Anal. calculated for $C_{15}H_{11}NO_2$: C, 75.94; H, 4.67; N, 5.90. Found: C, 76.14; H, 4.75; N, 6.01.

Example 11 2-(4-acetylphenyl)benzoxazole, (Table 1 compound 9) (Method B)

As described above, o-aminophenol (873 mg, 8.00 mmol), 4-bromoacetophenone (1.592 g, 8.00 mmol), $PdCl_2L_2$ (84 mg, 0.12 mmol), $PPh_3$ (63 mg, 0.25 mmol), toluene (25 mL) and DBU (1.44 mL, 9.60 mmol) were allowed to react together for 3 h under 7.73 kg/sq.cm CO then transferred to a flask, diluted with toluene and heated to reflux in the presence of p-toluenesulfonic acid (p-TSA, 6.0 g) for 2 h. After extraction with hot toluene, the toluene fractions were combined, concentrated in vacuo and the solid recrystallized from toluene to give 1.63 g product (86%).

Example 12 2-(4-nitrophenyl)benzoxazole, (Table 1 compound 10) (Method B)

As described above, o-aminophenol (873 mg, 8.00 mmol), 4-nitroiodobenzene (1.992 g, 8.00 mmol), $PdCl_2L_2$ (84 mg, 0.12 mmol), toluene (25 mL) and DBU (1.44 mL, 9.60 mmol) were allowed to react together for 20 min under 7.73 kg/sq.cm CO then transferred to a flask, diluted with toluene and heated to reflux in the presence of p-toluenesulfonic acid (p-TSA, 6.0 g) for 2 h. After treatment with $NH_4OH$, the solution was concentrated and allowed to stand overnight. 1.42 g (74%) product was obtained as light brown solid. mp 257 - 260 °C. literature mp 263 °C. IR(KBr) 3100, 1600, 1560, 1520, 1450, 1340, 1105, 1055, 855, 750, 705 $cm^{-1}$.

Example 13 6-Methyl-2-phenylbenzoxazole (Table 1 compound 11) (Method B)

As described above, 6-amino-m-cresol (1.101 g, 8.94 mmol), iodobenzene (1.00 mL, 8.94 mmol), $PdCl_2L_2$(94 mg, 0.13 mmol), toluene (27 mL) and DBU (1.60 mL, 10.07 mmol) were allowed to react together for 1.5 h under 7.73 kg/sq. cm CO then transferred to a flask, diluted with toluene and heated to reflux in the presence of p-toluenesulfonic acid (p-TSA, 6.0 g) for 2 h. The usual workup gave 1.66 g product (88%). mp 92 - 93 °C. literature mp 92 - 93 °C. 1H NMR ($CDCl_3$) δ 8.22 (m, 2), 7.63 (d, J = 8.1 Hz, 1), 7,48 (m, 3), 7.33 (br s , 1), 7.13 (d, J = 7.9 Hz, 1), 2.46 (s, 3). 13C{1H} NMR ($CDCl_3$) d 162.4, 150.9, 139.8, 135.4, 131.1, 128.7, 127.3, 127.2, 125.6, 119.2, 110.6, 21.6. IR(KBr) 3055, 2920, 2860, 1615, 1555, 1480, 1455, 1335, 1245, 1050, 1020, 810, 770, 700, 690 cm-1.

Example 14 5-Methyl-2-phenylbenzoxazole (Table 1 compound 12) (Method B)

As described above, 2-amino-p-cresol (1.101 g, 8.94 mmol), iodobenzene (1.00 mL, 8.94 mmol), $PdCl_2L_2$ (94 mg, 0.13 mmol), toluene (27 mL) and DBU (1.60 mL, 10.07 mmol) were allowed to react together for 1.75 h under 7.73 kg/sq.cm CO then transferred to a flask, diluted with toluene and heated to reflux in the presence of p-toluenesulfonic acid (p-TSA, 6.0 g) for 2 h. The usual workup gave 1.59 g product (85%). mp 104-105 °C literature mp 103 -104 °C. 1H NMR ($CDCl_3$) δ 8.24 (m,2), 7.56 (s, 1), 7.51

(m, 3), 7.43 (d, J = 7.3 Hz, 1), 7.14 d, J = 8.0 Hz, 1), 2.48 (s, 3). 13C{1H} NMR ($CDCl_3$) d 162.1, 148.9, 142.2, 134.3, 131.3, 128.8, 127.4, 127.3, 126.1, 119.8, 109.8, 21.5. IR(KBr) 3055, 2910, 2855, 1550, 1475, 1445, 1335, 1255, 1200, 1055, 1020, 925, 820, 795, 775, 700, 690 $cm^{-1}$.

Example 15 5,7-Dimethyl-2-phenylbenzoxazole (Table 1 compound 13) (Method B)

As described above, 2-amino-4,6-dimethylphenol (1.226 g, 8.94 mmol), iodobenzene (1.00 mL, 8.94 mmol), $PdCl_2L_2$ (94 mg, 0.13 mmol), toluene (27 mL) and DBU (1.60 mL, 10.07 mmol) were allowed to react together for 2 h under 7.73 kg/sq.cm CO then transferred to a flask, diluted with toluene and heated to reflux in the presence of p-toluenesulfonic acid (p-TSA, 6.0 g) for 2 h. After extracting with hot toluene, the organic solution was concentrated and cooled. The solid which crystallized was washed with cold toluene and dried to give 438 mg (22%) pure product. The liquors were subjected to the usual workup and gave 1.17 g product (59%). Purification (3:1, hexane:EtOAc) gave product with mp 98.5 - 100°C. 1H NMR ($CDCl_3$) $\delta$ 8.25 (m, 2), 7.50 (m, 3), 7.38 (s, 1), 6.94 (s, 1), 2.53 (s, 3), 2.44 (s, 3). 13C{1H} NMR ($CDCl_3$) d 162.6, 148.1, 141.8, 134.1, 131.1, 128.7, 127.4, 127.3, 120.3, 117.1, 21.4, 15.1. IR(KBr) 3055, 2920, 2860, 1555, 1480, 1450, 1335, 1190, 1100, 1055, 1020, 820, 795, 700, 690 $cm^{-1}$. Anal. calculated for $C_{15}H_{13}NO$: C, 80.69; H, 5.87; N, 6.27. Found C: 80.61; H, 5.89; N, 6.16.

Example 16 2-Phenylnapth[2,3-d]oxazole, (Table 1 compound 14) (Method B)

As described above, 3-amino-2-naphthol (1.139 g, 7.15 mmol), iodobenzene (800 mL, 8.94 mmol), $PdCl_2L_2$ (76 mg, 0.11 mmol), toluene (22 mL) and DBU (1.28 mL, 8.56 mmol) were allowed to react together for 2 h under 7.73 kg/sq.cm CO then transferred to a flask, diluted with toluene and heated to reflux in the presence of p-toluenesulfonic acid (p-TSA, 6.0 g) for 2 h. After concentrating the toluene extracts the solid was recrystallized from DMAc/toluene mixture and washed with toluene and then with a hexane/EtOAc mixture to give 1.60 g (91%) product. mp 199 -200°C. literature mp 211-212°C. 1H NMR ($CDCl_3$) $\delta$ 8.34 (m, 2), 8.20 (s, 1), 7.98 (m, 2), 7.94 (s, 1), 7.57 (m, 3), 7.49 (m, 2). 13C{1H} NMR ($CDCl_3$) $\delta$ 164.9, 149.6, 142.0, 132.0, 131.7, 131.5, 128.9, 128.4, 128.0, 126.8, 125.4, 124.6, 117.2, 106.3. IR(KBr) 3055, 1620, 1555, 1440, 1410, 1320, 1245, 1050, 1020, 860, 780, 750, 700 $cm^{-1}$.

Example 17 5-Chloro-2-phenylbenzoxazole (Table 1 compound 15) (Method B)

As described above, 5-chloro-2-aminophenol (1.284 g, 8.94 mmol), iodobenzene (1.00 mL, 8.94 mmol), $PdCl_2L_2$ (94 mg, 0.13 mmol), toluene (27 mL) and DBU (1.60 mL, 10.07 mmol) were allowed to react together for 1h under 7.73 kg/sq.cm CO then transferred to a flask, diluted with toluene and heated to reflux in the presence of methanesulfonic acid (2.0 mL) for 2.5 h. More methanesulfonic acid (1.0 mL) was added and the reaction allowed to continue for another 2 h. The usual workup gave 2.00 g crude product (98%). An impurity was sublimed away from the crude product (80°C/0.17 torr) to give 1.55 g (79%) pure product. mp 103-104°C. literature mp 100-101°C. 1H NMR ($CDCl_3$) $\delta$ 8.20 (dd, J = 7.5, 2.2 Hz, 2), 7.72 (d, J = 8.7 Hz, 1), 7.50 (m, 3), 7.45 (d, J = 8.7 Hz, 1), 7.28 (dd, J = 8.6, 2.0 Hz,1). 13C(1H) NMR ($CDCl_3$) d 164.2, 149.2, 143.2, 131.8, 129.9, 128.9, 127.7, 126.6, 125.2, 119.9, 111.2. IR(KBr) 3065, 1610, 1550, 1445, 1335, 1265, 1055, 1025, 920, 865, 810, 700, 680 $cm^{-1}$.

General Procedure for Benzimidazoles and Benzthiazoles

Reactions were run in a pressure vessel as described in the general procedure for benzoxazole reactions.

Example 18 2-phenylbenzthiazole (Table 2 compound 21)

A Fischer-Porter bottle was charged with o-aminobenzenethiol (1.07 mL, 10.0 mmol), iodobenzene (800 µl, 7.16 mmol), $PdCl_2L_2$ (76 mg, 0.106 mmol), $PPh_3$ (56 mg, 0.212 mmol), DMAc (20 mL) and DBU (1.28 mL, 8.56 mmol) as described above, pressurized with 7.73 kg/sq.cm CO and allowed to react for 5 hours at 120°C. The CO pressure was released and the temperature raised to 170°C. After 19.5 hours the reaction mixture was concentrated to about 1 mL and then dissolved in ether. The insoluble material was removed by filtration and the ether filtrate concentrated in vacuo and purified on a 4 mm silica gel plate using 4:1 hexane:ethyl acetate to give 185 mg (12%) product. Sublimation at 120°C/0.05 torr gave white needles with mp 112-113°C. lit mp 111-113°C. 1H NMR($CDCl_3$) $\delta$ 7.40 (dt, J=1.0, 7.1Hz, 1), 7.50 (m, 4), 7.92 (d,

J=8.0Hz, 1), 8.12 (m, 3). IR(KBr) 3065, 3020, 1480, 1435, 1315, 1225, 965, 765, 690 $cm^{-1}$.

Example 19 Preparation of 2-phenylbenzimidazole (Table 2 compound 16)

A pressure reaction vessel was charged with o-diaminobenzene (967 mg, 8.94 mmol), iodobenzene (1.00 mL, 8.94 mmol), $PdCl_2L_2$ (94 mg, 0.13 mmol) and DMAc (27 mL). The contents were deoxygenated with argon then placed under 2.46 kg/sq.cm CO and stirred at 120° C until all reagents had dissolved. Then the pressure was released and 2,6-lutidine (1.40 mL, 12.0 mmol) was added by syringe through the ball valve. The reactor was charged to 7.73 kg/sq.cm CO and stirred for 19 h at 145° C. The reaction mixture was filtered through filter-aid to remove palladium and then concentrated in vacuo. The residue was dissolved in hot water, filtered hot, cooled and the product was collected as off-white crystals. The product was then slurried in water/ammonium hydroxide solution, filtered, dried in air and recrystallized from ethanol/water to give 1.4 g product (75 %). mp 289-291° C. 1H NMR ($CDCl_3$) $\delta$ 7.94 (dd, J = 7.8, 1.3 Hz, 2), 7.48 (m, 1), 7.22 (m, 4), 6.96 (m, 2).

Example 20 2-(4-cyanophenyl)benzimidazole (Table 2 compound 19)

As described above, o-diaminobenzene (967 mg, 8.94 mmol), 4-cyanoiodobenzene (2.047g, 8.94 mmol), $PdCl_2L_2$ (94 mg, 0.13 mmol), 2,6-lutidene (1.40 mL, 12.0 mmol) and DMAc (27 mL) were allowed to react under 7.73 kg/sq.cm CO at 145° C for 24 h. After filtering the reaction mixture and concentrating the filtrate, the residue was recrystallized from 50% aqueous acetic acid then slurried in water/ammonium hydroxide solution, filtered, dried in air and recrystallized from ethanol/water to give 1.45 g product (74%). mp 261-262° C. 1H NMR (DMSO-$d_6$) $\delta$ 13.17 (br s, 1), 8.30 (d, J = 8.4 Hz, 2), 7.95 (d, J = 8.2 Hz, 2), 7.61 (br s, 2), 7.21 (m, 2).

Example 21 2-(4-chlorophenyl)benzimidazole (Table 2 compound 18)

As described above, o-diaminobenzene (967 mg, 8.94 mmol), 4-chloroiodobenzene (2.132g, 8.94 mmol), $PdCl_2L_2$ (94 mg, 0.13 mmol), 2,6-lutidene (1.40 mL, 12.0 mmol) and DMAc (27 mL) were allowed to react under 7.73 kg/sq.cm CO at 145° C for 24 h. After filtering the reaction mixture and concentrating the filtrate, the residue was recrystallized from 50% aqueous acetic acid to give 2.09 g product. This was then slurried in water/ammonium hydroxide solution, filtered, dried in air and recrystallized from ethanol/water to give 1.90 g product (93%). mp 290-292° C. 1H NMR (DMSO-$d_6$) $\delta$ 12.98 (br s, 1), 8.18 (d, J = 8.4 Hz, 2), 7.58 (d, J = 8.4 Hz, 2), 7.57 (m, 2), 7.19 (m, 2).

Example 22 2-(4-methylphenyl)benzimidazole (Table 2 compound 17)

As described above, o-diaminobenzene (967 mg, 8.94 mmol), 4-iodotoluene (1.949 g, 8.94 mmol), $PdCl_2L_2$ (94 mg, 0.13 mmol), 2,6-lutidene (1.40 mL, 12.0 mmol) and DMAc (27 mL) were allowed to react under 7.73 kg/sq.cm CO at 145° C for 24 h. After filtering the reaction mixture and concentrating the filtrate, the residue was recrystallized from 50% aqueous acetic acid then slurried in water/ammonium hydroxide solution, filtered, dried in air and recrystallized from ethanol/water to give 1.50 g product (81%). mp 270-272° C. 1H NMR (DMSO-$d_6$) $\delta$ 12.80 (br s, 1), 8.05 (d, J = 8.0 Hz, 2), 7.56 (m, 2), 7.32 (d, J = 8.0 Hz, 2), 7.16 (m, 2), 2.33 (s, 3).

Example 23 2-phenylnaphthimidazole (Table 2 compound 20)

As described above, 2,3-diaminonaphthalene (1.414 g, 8.94 mmol), iodobenzene (1.00 mL, 8.94 mmol), $PdCl_2L_2$ (94 mg, 0.13 mmol), 2,6-lutidene (1.40 mL, 12.0 mmol) and DMAc (27 mL) were allowed to react under 7.73 kg/sq.cm CO at 145° C for 24 h. After filtering the reaction mixture and concentrating the filtrate, the residue was recrystallized from 50% aqueous acetic acid then slurried in water/ammonium hydroxide solution, filtered, dried in air and recrystallized from ethanol/water to give 1.67 g product (77%). mp 210-212° C. 1H NMR (DMSO-$d_6$) $\delta$ 12.96 (br s, 1), 8.27 (d, J = 6.7 Hz, 2), 8.09 (m, 2), 7.98 (m, 2), 7.55 (m, 3), 7.34 (m, 2).

The present invention has been described with reference to particularly preferred embodiments thereof. However, it will be understood that modifications and extensions can be effected within the spirit and scope of the invention.

## Claims

1. A method for the production of heterocyclic organic compounds comprising the steps of:
   a) condensing an aromatic halide with an orthoamino(phenol, aniline, thiophenol) in the presence of carbon monoxide and a palladium catalyst; and
   b) causing the intermediate formed in step a) to undergo ring closure to give the desired heterocycle.

2. The method of claim 1 further comprising the step of neutralizing by-product hydrogen halide.

3. The method of claim 1 wherein said condensing step is conducted in the presence of base.

4. The method of claim 1, 2 or 3 wherein said aromatic halide is represented by the formula:

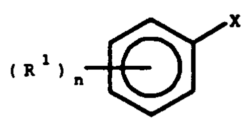

wherein X is Bromide or iodide;
$R^1$ is selected from the group consisting of alkyl, alkoxy, aryloxy, disubstituted amino, cyano, chloro, aryl, acetyl, nitro, carboxylic acid (and derivatives such as esters), sulfone and amide; and
n is an integer from 0 to 4.

5. The method of claim 1, 2, 3, or 4 wherein said ortho-amino(phenol, aniline, thiophenol) is represented by the formula:

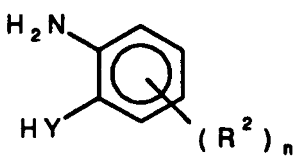

wherein Y is selected from the group consisting of O, NH and S;
$R^2$ is selected from the group consisting of alkyl, alkoxy, aryloxy, disubstituted amino, cyano, chloro, aryl, acetyl, nitro, carboxylic acid (and derivatives such as esters), sulfone and amide; and
n is an integer from 0 to 4.

6. The method of claim 1, 2, 3, 4 or 5 wherein said ring closure is accomplished by adding methanesulfonic or para-toluenesulfonic acid and refluxing to remove water.

7. The method of claim 1, 2, 3, 4 or 5 wherein said catalyst is $PdCl_2L_2$.

European Patent Office

# EUROPEAN SEARCH REPORT

Application Number

**EP 91 11 6559**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 053 291 (AMERICAN CYANAMID COMPANY)<br>* claims * *<br>– – – | 1-7 | C 07 D 263/56<br>C 07 D 277/66<br>C 07 D 235/18<br>C 07 D 263/60<br>C 07 D 413/04 |
| A | US-A-3 452 036 (H.P.CROCKER)<br>* the whole document * *<br>– – – | 1 | |
| A | DE-A-2 619 547 (DYNAMIT NOBEL A.G.)<br>* claims * *<br>– – – – – | 1 | |
| | | | **TECHNICAL FIELDS SEARCHED (Int. Cl.5)** |
| | | | C 07 D |

The present search report has been drawn up for all claims

| Place of search | Date of completion of search | Examiner |
|---|---|---|
| The Hague | 12 December 91 | HENRY J.C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document